# EUROPEAN PATENT SPECIFICATION

(11) **EP 3 505 093 B1**
(45) Date of publication and mention of the grant of the patent: **22.07.2020**
(21) Application number: 17210987.8
(22) Date of filing: 28.12.2017
(51) Int. Cl.: A61B 17/30, A61B 17/04, A61B 90/00, A61B 17/00, A61B 17/3207

(54) **MULTIFUNCTIONAL ANATOMICAL TWEEZERS**
CHIRURGISCHES WERKZEUG
OUTIL CHIRURGICAL

(43) Date of publication of application: 03.07.2019
(73) Proprietor: Sottile, Sebastiano, 21050 Porto Ceresio (Varese) (IT); Chmura, Marzena Iwona, 20150 Porto Ceresio (Varese) (IT)
(72) Inventor: SOTTILE, Sebastiano, I - 21050 PORTO CERESIO (Varese) (IT)
(74) Representative: Lunati & Mazzoni S.r.L.

(56) References cited:
- CN-U- 205 359 539
- GB-A- 2 383 952
- US-A- 4 452 106

## Description

The present invention relates to multifunctional anatomical tweezers of the type specified in the preamble of the first claim.

As is known, the dressing of lesions or wounds may involve the use of a plurality of instruments.

In the medical as well as the domestic fields, anatomical tweezers, scalpels, scissors and other objects can be used as required.

For example, when in the presence of sutures, it may be necessary to act on cutaneous tissues with both anatomical tweezers and scissors. In fact, it may be necessary to cut the suture thread and further intervene with a curette, which is substantially a spoon for medical use suitable for cleaning wounds.

The described prior art has a few major drawbacks.

In particular, an operator is forced to have both his/her hands busy if he/she needs, for example, to act on a suture.

Moreover, he/she is forced to use a plurality of instruments and therefore the medical activity procedures extend over time and get complicated.

However, devices that overcome some of the above mentioned drawbacks are known.

Documents US-A-4478221, CN-U-000205359539, US-A-3576072 and CN-U-000204863386 provide tweezers devices comprising retractable blades suitable to allow, for example, the cutting of a suture.

However, the devices of the aforementioned documents also have some major drawbacks.

They include means that are most likely to poke out in an inconvenient and dangerous manner for an operator who must often perform the operations with extreme speed.

Furthermore, the cutting means are sometimes external and may cause injury to the operators themselves with very serious consequences in the medical field.

In addition, all extraction systems are difficult to handle and the tweezers described do not allow all types of necessary operations to be performed essentially in the operating scope.

In this context, the technical task underlying the present invention is to devise multifunctional anatomical tweezers capable of substantially obviating at least some of the above-mentioned drawbacks.

Within the scope of said technical task, a major object of the invention is to obtain anatomical tweezers capable of carrying out a plurality of functions.

Another important object of the invention is to provide anatomical tweezers, which can facilitate some medical activities, such as for example suture operations, thus reducing the time required to carry out the same activities.

The technical task and the specified objects are achieved by means of multifunctional anatomical tweezers as claimed in the appended claim 1. Preferred embodiments are described in the dependent claims.

The features and advantages of the invention will be apparent from the detailed description of preferred embodiments of the invention, with reference to the accompanying drawings, in which:

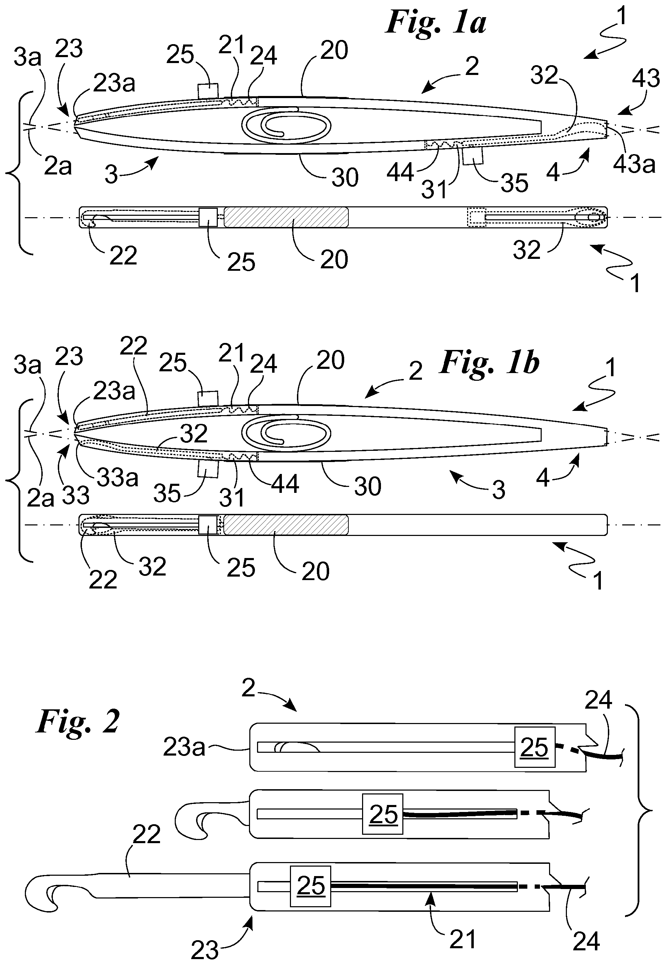

**Fig. 1a** shows a side view and a top view of the tweezers according to the invention in a preferred configuration;
**Fig. 1b** shows a side view and a top view of the tweezers according to the invention in an alternative configuration;
**Fig. 2** illustrates a portion and the operation of tweezers comprising a thread cutter according to the invention;

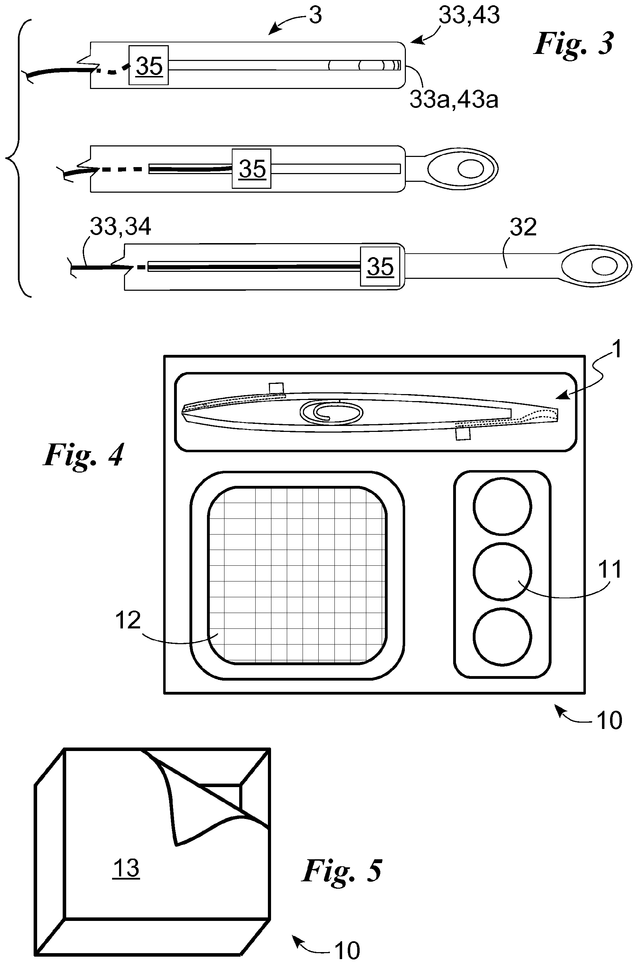

**Fig. 3** illustrates a portion and the operation of tweezers comprising a curette according to the invention;
**Fig. 4** shows an exemplary surgical kit comprising the tweezers according to the invention; and
**Fig. 5** shows the casing of the surgical kit in use for waste.

In the present document, the measures, values, shapes and geometric references (such as perpendicularity and parallelism), when associated with terms like "about" or other similar terms such as "almost" or "substantially", are to be understood as unless measurement errors or inaccuracies due to production and/or manufacturing defects and, especially, unless a slight difference from the value, measure, shape, or geometric reference with which it is associated. For example, these terms, if associated with a value, preferably indicate a difference not exceeding 10% of the value itself.

Furthermore, when used, terms such as "first", "second", "higher", "lower", "main" and "secondary" do not necessarily identify an order, a priority relationship or a relative position, but can simply be used to distinguish more clearly the different components from each other.

The measurements and the data reported in this text are to be considered, unless otherwise indicated, as carried out in the International Standard Atmosphere ICAO (ISO 2533).

With reference to the Figures, the multifunctional anatomical tweezers according to the invention are indicated as a whole with number **1.**

The multifunctional anatomical tweezers 1 preferably comprise at least a first rod **2** and a second rod **3.**

Preferably, the rods 2,3 are of the conventional type and therefore are adapted to bend to allow said tweezers 1 to grip an external object.

Such rods 2,3 may therefore comprise any kind of material provided it has elasticity. They may be made of metal, wood or other materials. Preferably, they are made of a polymeric material.

The first rod 2 and the second rod 3 further define a junction portion **4.**

The junction portion 4 is preferably the portion at which the rods 2,3 are mutually constrained.

In addition, the rods 2,3 define a first direction **2a** and a second direction **3a,** respectively.

The first and the second direction 2a,3a represent the trajectories described by the rods 2,3, respectively.

The latter may be of various kinds: curvilinear, horizontal or similar.

Directions 2a,3a preferably represent the midline described by the rods 2,3.

The first rod 2 comprises at least a first gripping portion **20.**

The first gripping portion 20 is a portion of the rod 2 defining a rough or knurled surface so as to provide the friction required to ensure a firm grip of the tweezers 1. Preferably, the second rod 3 also comprises a second gripping portion 30.

This second gripping portion 30 has the same characteristics as the first gripping portion 20.

The first rod 2 further comprises a first cavity **21.**

The first cavity 21 is located inside the first rod 2 and is preferably adapted to communicate with the outside along at least one opening located in the rod 2.

Likewise, the second rod 3 comprises a second cavity **31.**

The second cavity 31 is located inside the second rod 3 and is preferably adapted to communicate with the outside along at least one opening located in the rod 3. Preferably, the first rod 2 and the second rod 3 define, respectively, a first end **23** and a second end **33.**

The two ends 23,33 are adapted to cooperate with each other so as to allow the tweezers 1 to grip an external object.

Therefore, preferably, the two ends 23,33 are arranged on the opposite side of the junction portion 4.

More specifically, the first end 23 preferably defines a first opening **23a.**

The first opening 23a is preferably a hole located on the surface of the rod 2 at the first end 23.

Preferably, the opening 23a is adapted to allow the first cavity 21 to communicate with the outside.

The second end 33 may also define a second opening **33a.**

The second opening 33a, if present, is a hole located on the surface of the rod 3 at the second end 33.

Preferably, the second opening 33a is adapted to allow the second cavity 31 to communicate with the outside.

However, preferably, the second rod 3 defines a third end **43.**

The third end 43 is preferably opposite to the second end 33. Therefore, it is also opposite to the first end 23 and is substantially the end corresponding to the area occupied by the junction portion 4.

Preferably, the third end 43 defines a third opening **43a.**

The third opening 43a is a hole located on the surface of the rod 3 at the third end 43.

Preferably, the third opening 43a is also adapted to allow the second cavity 31 to communicate with the outside.

The third opening 43a, as described, may alternatively or in addition allow the first cavity 21 to communicate with the outside.

Therefore, the tweezers 1 enable a plurality of configurations in which the cavities 21,31 are arranged in different positions.

Furthermore, two first cavities 21 and/or two second cavities 31 may also be present.

In a preferred configuration, the tweezers 1 comprise a first cavity 21 adapted to communicate with the outside through the first opening 23a, and a second cavity 31 adapted to communicate with the outside through the third opening 43a.

In this manner, the two openings 23a,43a are at opposite ends.

The first cavity 21 further comprises a first slider **22.**

The first slider 22 is a slider of the retractable type and, therefore, is preferably adapted to be housed inside the cavity 21 and, optionally, to protrude at least partially therefrom.

The first slider 22 is preferably adapted to emerge on command through the first opening 23a of the end 23 from the first cavity 21.

This slider 22 is preferably adapted to emerge from the cavity 21 in opposition to a first elastic member **24.**

The first elastic member 24 can be a spring, a rubberized thread or other elements suitable to allow a force to be exerted in opposition to the movement of the slider 22 outside the first cavity 21.

Preferably, the first slider 22 therefore includes a thread cutter.

The thread cutter consists of a cutting blade suitable for cutting the suture thread or fibrin edges or filaments.

The second cavity 31 preferably comprises a second slider **32.**

The second slider 22 is a slider of the retractable type and, therefore, is preferably adapted to be housed inside the second cavity 31 and, optionally, to protrude at least partially therefrom.

The second slider 22 is preferably adapted to emerge on command through the third opening 43a of the third end 43 from the second cavity 31.

This second slider 32 is preferably adapted to emerge from the second cavity 31 in opposition to a third elastic member **44.**

The third elastic member 44 can also be a spring, a rubberized thread or other elements suitable to allow a force to be exerted in opposition to the movement of the slider 32 outside the second cavity 31.

In an alternative configuration of the tweezers 1, however, the second slider 32 may emerge on command through the second opening 33a of the second end 33 from the second cavity 31.

In this case, the second slider 32 is adapted to emerge from the second cavity 31 in opposition to a second elastic member **34.**

The second elastic member 34 has the same characteristics as the other elastic members 24,44.

Preferably, the second slider 32 therefore includes a curette.

The curette consists of a surgical spoon for removing fibrin, also known as the debridement operation, and therefore allows wound cleansing.

The two sliders 22,32 can be made of different materials.

Preferably, they are made of metal.

In addition, the sliders 22, 32 comprise blades suitable to emerge on command by means of a first lever **25** and a second lever **35,** respectively.

The levers 25,35 are preferably manually operated and accessible at the surface of the rods 2,3.

Preferably, moreover, the first cavity 21 defines a guide along the first direction 2a for the first slider 22, and the second cavity 31 defines a guide along the second direction 3a for the second slider 32.

Therefore, the levers 25,35 are knobs suitable to allow the movement of the sliders 22,32 along directions 2a,3a, respectively.

The tweezers 1 may be included within a surgical kit **10.**

The surgical kit 10 preferably comprises the tweezers 1, dressing means **12** and sanitizing means **11.**

The dressing means 12 preferably comprise a plurality of sachets containing medical substances.

These medical substances can be selected from a detergent containing polyhexanide and betaine, betadine, a iodopovidone-based disinfectant.

The sanitizing means 11 preferably comprise a plurality of gauzes, cotton wads and at least one cloth.

Therefore, the surgical kit 10 preferably contains all the instruments required to perform an operation, for example, the removal of stitches.

The surgical kit 10 is also preferably disposable and comprises a package **13.** The package 13 is preferably sealed with a tear-off seal and it contains - and is adapted to allow a single use of - the tweezers 1, the dressing means 12 and the sanitizing means 11.

In one example, the surgical kit 10 contains three sachets, of which one with Prontosan (polyhexanide and betaine), one with betadine (antiseptic solution for the treatment of small wounds and skin infections) or braunol (iodopovidone-based disinfectant), one sachet with 0.9% NaCI (sodium chloride), five 3x3 cm disposable gauze veils, three wads and lastly a 15x15 cm folded cloth which is used for the dressing.

The package 13 may have two closures. One preferably closes the whole surgical kit 10 package, the other separates the dressing means 12 therein. Once opened, the latter can also be used as a waste bag. The package 13 can have reduced dimensions, a depth of about 15 mm and a width of 13x13 cm, useful for in-home operations.

The operation of the multifunctional anatomical tweezers 1, previously described in structural terms, is as follows.

The tweezers 1 allow normal operation of tweezers to be performed, but if necessary they make it possible to extract the thread cutter therefrom for cutting a thread, for example a suture thread, and to cleanse any wounds with the curette.

These means are extractable in opposition to an elastic member by means of the levers, which can be operated manually with a finger of the hand gripping the tweezers. The internal mechanisms, defined by the elastic members 24,34,44, allow the sliders 22,32 to be extracted and to be automatically brought back into the cavities 21,31 when the command imparted by the finger is removed.

The multifunctional anatomical tweezers 1 according to the invention achieve important advantages.

In fact, they can be used for the removal of suturing stitches in hollow areas such as inside the mouth, or in the presence of bedsores with cavernous necrosis with suturing stitches, as well as in other operations, enabling a user to avoid replacement of the instrument.

The tweezers 1 substantially contain everything needed to perform the operation in its entirety.

In addition, they enable greater manoeuvrability compared to what is known, since they are easily usable with one hand.

The fact that they comprise retractable elements therefore allows handiness to be combined with extreme ease of use and safety.

In fact, the ergonomics of the tweezers is in no way compromised, and no blade or other instrument can cut the hands of the operator using the tweezers 1.

In conclusion, the tweezers 1 comprised within a surgical kit, as described, allow, for example, operations also to be performed at home where usually two hands are not sufficient.

The tweezers 1 that are operated with one hand allow the other hand to be free, which can therefore be used by the operator, thus making his/her healthcare activities easier and avoiding any inconvenience for the user or increasing his/her comfort.

The invention is susceptible of variations falling within the scope of the inventive concept as defined by the claims.

In this context, all details are replaceable by equivalent elements and the materials, shapes and dimensions may be any materials, shapes and dimensions.

## Claims

1. A multifunctional anatomical tweezers (1) comprising:
- a first rod (2) and a second rod (3) including, respectively, at least a first gripping area (20) and a second gripping area (30),
- said first rod (2) comprising at least a first cavity (21),
- said first cavity (21) comprising a first retractable slider (22) including a thread cutter,
and said tweezers (1) being **characterised in that**
- said second rod (3) comprises a second cavity (31),
- said second cavity (31) comprises a second retractable slider (32) including a curette.

2. The tweezers (1) according to claim 1, wherein said first rod (2) defines a first end (23) and said second rod defines a second end (33),
said ends (23, 33) being adapted to allow said tweezers (1) to grip an external object, and said first end (23) defining a first opening (23a) for said first cavity (21).

3. The tweezers (1) according to at least one of the preceding claims, wherein said second end (23) defines a second opening (33a) for said second cavity (31).

4. The tweezers (1) according to at least one of the preceding claims, wherein said rods (2,3) define a junction portion (4) at which said rods (2,3) are mutually constrained, and said second rod (3) defines a third end (43) opposite to said second end (33), defining a third opening (43a) for said second cavity (21) and located at said junction portion (4).

5. The tweezers (1) according to at least one of the preceding claims, wherein said first slider (22) is adapted to emerge on command through said first opening (23a) of said first end (23) from said first cavity (21) in opposition to a first elastic member (24).

6. The tweezers (1) according to at least one of the preceding claims, wherein said second slider (32) is adapted to emerge on command through an opening selected from said second opening (33a) of said second end (33) from said second cavity (31) in opposition to a second elastic member (34) and said third opening (43a) of said third end (43) from said second cavity (31) in opposition to a third elastic member (44).

7. The tweezers according to at least one of the preceding claims, wherein said sliders (22, 32) comprise blades suitable to emerge on command by means of a first lever (25) and a second lever (35), respectively, which are manually operated and accessible at the surface of said rods (2,3).

8. The tweezers according to at least one of the preceding claims, wherein said rods (2,3) define a first direction (2a) and a second direction (3a), respectively, said first cavity (21) defines a guide along said first direction (2a) for said first slider (22), and said second cavity (31) defines a guide along said second direction (3a) for said second slider (32), and said levers (25,35) are knobs suitable to allow the movement of said sliders (22,32) along said directions (2a,3a), respectively.

9. A surgical kit (10) comprising tweezers (1) according to at least one of the preceding claims, dressing means (12) comprising a plurality of sachets (12) containing medical substances, and sanitizing means (11) comprising a plurality of gauzes, cotton wads and at least one cloth.

10. The surgical kit (10) according to at least one of the preceding claims, wherein said sachets contain substances selected from a detergent containing polyhexanide and betaine, betadine and a iodopovidone-based disinfectant, and said surgical kit (10) is disposable and comprises a package (13) sealed with a tear-off seal and containing - and adapted to allow a single use - of said tweezers (1), said dressing means (12) and said sanitizing means (11).

## Patentansprüche

1. Chirurgisches Werkzeug (1), umfassend:
- einen ersten Bügel (2) und einen zweiten Bügel (3), die jeweils mindestens einen ersten Griffbereich (20) und einen zweiten Griffbereich (30) umfassen,
- wobei der genannte erste Bügel (2) mindestens einen ersten Hohlraum (21) umfasst,
- wobei der genannte erste Hohlraum (21) einen einziehbaren ersten Cursor (22) umfasst, der einen ersten Fadenschneider einschließt,
und wobei das genannte chirurgische Werkzeug (1) **dadurch gekennzeichnet ist, dass**
- der genannte zweite Bügel (3) einen zweiten Hohlraum (31) umfasst,
- der genannte zweite Hohlraum (31) einen einziehbaren zweiten Cursor (32) umfasst, der eine Kürette einschließt,

2. Chirurgisches Werkzeug (1) nach Anspruch 1, bei dem der genannte erste Bügel (2) ein erstes Ende (23) definiert und der genannte zweite Bügel ein zweites Ende (33) definiert,
wobei die genannten Enden (23, 33) geeignet sind, das Greifen eines externen Gegenstands von Seiten des genannten chirurgischen Werkzeugs (1) zu gestatten und das genannte erste Ende (23) eine erste Öffnung (23a) für den genannten ersten Hohlraum (21) definiert.

3. Chirurgisches Werkzeug (1) nach mindestens einem der vorangegangenen Ansprüche, bei dem das genannte zweite Ende (23) eine zweite Öffnung (33a) für den genannten zweiten Hohlraum (31) definiert.

4. Chirurgisches Werkzeug (1) nach mindestens einem der vorangegangenen Ansprüche, bei dem die genannten Bügel (2,3) einen Verbindungsabschnitt (4) definieren, auf dem die genannten Bügel (2,3) miteinander verbunden sind und der genannte zweite Bügel (3) ein dem genannten zweiten Ende (33) gegenüberliegendes drittes Ende (43) definiert, das eine dritte Öffnung (43a) für den genannten zweiten Hohlraum (21) definiert und auf dem genannten Verbindungsabschnitt (4) angeordnet ist.

5. Chirurgisches Werkzeug (1) nach mindestens einem der vorangegangenen Ansprüche, bei dem der genannte erste Cursor (22) geeignet ist, auf Befehl über die genannte erste Öffnung (23a) des genannten ersten Endes (23) aus dem genannten ersten Hohlraum (21) gegen den Widerstand eines ersten Federelements (24) auszutreten.

6. Chirurgisches Werkzeug (1) nach mindestens einem der vorangegangenen Ansprüche, bei dem der genannte zweite Cursor (32) geeignet ist, auf Befehl über wahlweise die genannte zweite Öffnung (33a) des genannten zweiten Endes (33) aus dem genannten zweiten Hohlraum (31) gegen den Widerstand eines zweiten Federelements (34) und die genannte dritte Öffnung (43a) des genannten dritten Endes (43) aus dem genannten zweiten Hohlraum (31) gegen den Widerstand eines dritten Federelements (44) auszutreten.

7. Chirurgisches Werkzeug nach mindestens einem der vorangegangenen Ansprüche, bei dem die genannten Cursoren (22, 32) Bleche umfassen, die geeignet sind, auf Befehl mittels jeweils eines ersten manuellen Hebels (25) und eines zweiten manuellen Hebels (35) auf der Oberfläche der genannten Bügel (2,3) auszutreten.

8. Chirurgisches Werkzeug nach mindestens einem der vorangegangenen Ansprüche, bei dem die genannten Bügel (2,3) jeweils eine erste Richtung (2a) und eine zweite Richtung (3a) definieren, der genannte erste Hohlraum (21) eine Führung entlang der genannten ersten Richtung (2a) für den genannten ersten Cursor (22) definiert und der genannte zweite Hohlraum (31) eine Führung entlang der genannten zweiten Richtung (3a) für den genannten zweiten Cursor (32) definiert und die genannten Hebel (25,35) aus Reglern bestehen, die geeignet sind, die Bewegung der genannten Cursoren (22,32) jeweils entlang der genannten Richtungen (2a,3a) zu gestatten.

9. Chirurgischer Kit (10), umfassend ein chirurgisches Werkzeug (1) nach mindestens einem der vorangegangenen Ansprüche, eine Vielzahl von medizinische Substanzen enthaltende Beutel (12) umfassendes Verbandszeug (12) und eine Vielzahl von Gazen, Wattebäusche und mindestens ein Tuch umfassende Hygienisierungsmittel (11).

10. Chirurgischer Kit (10) nach mindestens einem der vorangegangenen Ansprüche, in dem die genannten Beutel wahlweise ein Substanzen wie Polihexanid und Betain enthaltendes Reinigungsmittel, Betadine und ein Desinfektionsmittel aus Povidon-Jod enthalten und der genannte chirurgische Kit (10) ein versiegeltes aufreißbares Einwegprodukt (13) ist und das genannte chirurgische Werkzeug (1), das genannte Verbandszeug (12) und die genannten Hygienisierungsmittel (11) enthält und geeignet ist, ihren einmaligen Gebrauch zu gestatten.

## Revendications

1. Outil chirurgical (1) comprenant :
- une première tige (2) et une deuxième tige (3) incluant respectivement au moins une première zone de préhension (20) et une deuxième zone de préhension (30),
- ladite première tige (2) comprenant au moins une première cavité (21),
- ladite première cavité (21) comprenant un premier curseur (22) escamotable incluant un coupe-fil,
et ledit outil (1) étant **caractérisé en ce que**
- ladite deuxième tige (3) comprend une deuxième cavité (31),
- ladite deuxième cavité (31) comprenant un deuxième curseur (32) escamotable incluant une curette.

2. Outil (1) selon la revendication 1, dans lequel ladite première tige (2) définit une première extrémité (23) et ladite deuxième tige définit une deuxième extrémité (33),
lesdites extrémités (23, 33) étant aptes à permettre la préhension d'un objet externe au moyen dudit outil (1) et ladite première extrémité (23) définissant une première ouverture (23a) pour ladite première cavité (21).

3. Outil (1) selon au moins une revendication précédente, dans lequel ladite deuxième extrémité (23) définit une deuxième ouverture (33a) pour ladite deuxième cavité (31).

4. Outil (1) selon au moins une revendication précédente, dans lequel lesdites tiges (2, 3) définissent une partie de jonction (4) en correspondance de laquelle lesdites tiges (2, 3) sont réciproquement liées et ladite deuxième tige (3) définit une troisième extrémité (43) opposée à ladite deuxième extrémité (33), qui définit une troisième ouverture (43a) pour ladite deuxième cavité (21) et disposée en correspondance de ladite partie de jonction (4).

5. Outil (1) selon au moins une revendication précédente, dans lequel ledit premier curseur (22) est apte à émerger de façon commandée, à travers ladite première ouverture (23a) de ladite première extrémité (23), de ladite première cavité (21) en s'opposant à un premier élément élastique (24).

6. Outil (1) selon au moins une revendication précédente, dans lequel ledit deuxième curseur (32) est apte à émerger de façon commandée, à travers une au choix entre ladite deuxième ouverture (33a) de ladite deuxième extrémité (33) de ladite deuxième cavité (31) en s'opposant à un deuxième élément élastique (34) et ladite troisième ouverture (43a) de ladite troisième extrémité (43), de ladite deuxième cavité (31) en s'opposant à un troisième élément élastique (44).

7. Outil selon au moins une revendication précédente, dans lequel lesdits curseurs (22, 32) comprennent des lamelles aptes à émerger de façon commandée, respectivement, au moyen d'un premier levier (25) et d'un deuxième levier (35) manuels et accessibles sur la surface desdites tiges (2,3).

8. Outil selon au moins une revendication précédente, dans lequel lesdites tiges (2, 3) définissent respectivement une première direction (2a) et une deuxième direction (3a), ladite première cavité (21) définit un guidage le long de ladite première direction (2a) pour ledit premier curseur (22) et ladite deuxième cavité (31) définit un guidage le long de ladite deuxième direction (3a) pour ledit deuxième curseur (32), et lesdits leviers (25, 35) sont des poignées aptes à permettre le déplacement desdits curseurs (22, 32) respectivement le long desdites directions (2a, 3a).

9. Kit chirurgical (10) comprenant un outil (1) selon au moins une revendication précédente, des moyens de médication (12) comprenant une pluralité de sachets (12) contenant des substances médicales et des moyens d'hygiénisation (11) comprenant une pluralité de bandes de gazes, tampons de coton et au moins une toile.

10. Kit chirurgical (10) selon au moins une revendication précédente, dans lequel lesdits sachets contiennent des substances au choix parmi un détergent contenant du polihexanide et de la bétaïne, de la bétadine et un désinfectant à base de povidone iodée et ledit kit chirurgical (10) est jetable et comprend un emballage (13) scellé déchirable et apte à permettre un usage unique dudit outil (1), et contenant lesdits moyens de médication (12) et lesdits moyens d'hygiénisation (11).
